# EUROPEAN PATENT APPLICATION

(11) **EP 1 872 719 A1**
(43) Date of publication of application: **02.01.2008**
(21) Application number: 06013258.6
(22) Date of filing: 27.06.2006
(51) Int. Cl.: A61B 5/117, G06K 9/00, A61B 5/00

(54) **Liveness detection method**

(71) Applicant: TBS Holding AG, 8808 Pfäffikon SZ (CH)
(72) Inventor: Diaz-Santana, Eva, 8808 Pfäffikon (CH); Parziale, Giuseppe, 8808 Pfäffikon (CH)
(74) Representative: Liedtke, Markus

(57) **Abstract**

A liveness detection method, wherein at least one image (I) of a segment (1) of human or animal skin is captured by a touchless optical sensor and subjected to an analysis for detecting the perspiration activity of sweat pores.
Further disclosed is a device for applying said method, comprising at least a touchless optical sensor for capturing images (I) and an apparatus for detecting sweat pore perspiration activity in at least one of said images (I).

## Description

The invention relates to a liveness detection method and a device for applying it.

Biometric identification is based on the measurement of physiological and behavioral characteristics of an individual. In this sense, biometrics is the science of using a particular biological aspect of the human body to recognize a person for security, attendance or any other purpose for which proof of identity is required. The most widely used biometrics is fingerprints, but technological developments over the last decade have added DNA matching; iris and retinal scans; voice, handwriting, or facial recognition; and hand and facial geometry to the list of biometrics identifiers. Recent academic and media tests, however, show that with relatively modest effort many leading biometric systems are susceptible to attacks in which fake fingerprints, static facial images and static iris images can be used successfully as biometric samples. To overcome such spoofing attacks, liveness detection methods are needed. One method for detecting liveness suggests to detect perspiration in a time progression of fingerprint images captured with a capacitance scanner. For description see: Schuckers SAC, Spoofing and Anti-Spoofing Measures, Information Security Technical Report, Vol. 7, No. 4, pages 56-62, 2002. One problem is that only a rough estimate of local moisture due to perspiration can be detected because sweat emerging from pores is smeared over a platen touched by the finger. This makes difficult to locate the exact position of the sweating pores and impossible to track the perspiration activity.

It is accordingly an object of the invention to provide a novel method for liveness detection and a device for applying that method.

With that object in view, the present invention suggests a liveness detection method, wherein at least one image of a segment of human or animal skin is captured by a touchless optical sensor and subjected to an analysis for detecting the perspiration activity of sweat pores. Living skin has sweat pores, whose perspiration activity can be observed. Normally, the method will be applied to a finger or a palm. The optical sensor is required to be touchless so sweat can emerge from the pores freely, build droplets and be absorbed instead of being constricted or getting smeared by a platen or the respective surface of another scanner, that needs to be touched to operate. Sweat pore perspiration activity can distinguish a live finger, palm or other skinned part of the body from artificial, dismembered or cadaver body parts.

The analysis can comprise image segmentation, whereby the total image is segmented into sub-regions in order to ease the detection of features like sweating pores, sweat droplets emerging or droplets of lipid-soluble substances being absorbed within these regions. An emerging sweat droplet will typically cause a local change in colour or brightness, which can be easily detected.

According to a preferred embodiment of the invention a sequence of images is captured and analyzed. Thus different stages of the sweat pores activity are observable, e.g. by detecting a local change in colour or brightness from one image to a subsequent one.

According to another preferred embodiment of the invention said segment of skin is heated to create a challenge/response situation. With an increase in temperature, live skin is caused to perspire even more. By applying heat to the skin during capturing the image sequence the skins reaction to that heat can be checked, which is a very strong anti spoof measure.

For that purpose, a heater is arranged so it can heat an area within the range of the touchless optical sensor.

In a preferred embodiment of the invention the segment of skin is illuminated in order to take full advantage of the optical sensor's dynamic range and to avoid noise in the image. Different kinds of light sources can be used for that purpose, e.g. LED or fiber conductors with different spectral ranges (blue, green, yellow, red, infra red). Infra red light can serve for illumination and heating at the same time.

Especially in case the segment of skin is situated on a palm or finger it is possible to detect minutiae in the image in order to combine liveness detection with fingerprint recognition or enrolling. Minutiae are the points of interest in a fingerprint, such as bifurcations and ridge endings, which are unique for every individual.

The position of the sweat pores is unique and their detection can further improve fingerprint recognition and decrease false matching rate and false non matching rate, particularly because sweat pores are more difficult to fake in artificial fingers or palms due to their small size compared to ordinary fingerprint patterns. Sweat pores, once formed on the ridges in a late fetal stage, are fixed at that location for life. They do not disappear, move or spontaneously generate over time and can thus, be well used for recognition.

The method is preferably implemented in a liveness detection device, comprising at least a touchless optical sensor for capturing images and an apparatus for detecting sweat pore perspiration activity in at least one of said images. The optical sensor can be a camera or a system of cameras or any other sensor suited to capture images of appropriate resolution and with a distance to the object different from 0.

The apparatus for detecting sweat pore perspiration activity can be a computer, embedded system or the like which detection algorithms are implemented in.

In a preferred embodiment of the invention the touchless optical sensor is designed to capture 3D images. 3D images yield more useful information than standard 2D images. A 3D image can be used to detect sweat droplets not only by changes in colour or brightness but also by changes in shape and/or size of the droplets. When recognizing minutiae or the 3D positions of sweat pores, false matching rate and false non-matching rate can be improved dramatically compared to their recognition in a 2D image. When using a swept scanner 3D imaging can be achieved by using beam splitters and other optics to create more than one optical plane each equipped with a charge coupled device or the like. In order to detect fine details such as sweat pores the touchless optical sensor resolution has to be relatively high, e.g. 500 dpi or better.

A liveness detection device will now be described in detail with reference to the accompanying drawing, in which:

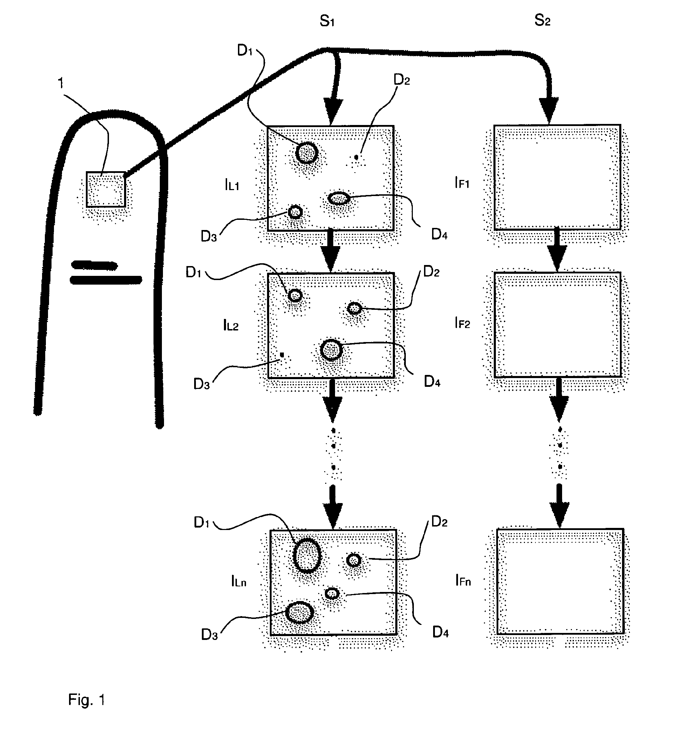

- Figure 1: is a diagram showing a finger and two sequences of images captured from a segment of the finger, depicting how sweat pore activity can be used to detect liveness

**Figure 1** shows a tip of a finger. The square represents a segment 1 of skin captured using a touchless optical sensor (not shown), which can be a camera, an arrangement of a multitude of cameras or a charge coupled device or the like swept over the segment 1. Typically the touchless optical sensor will capture a sequence of images I. The left column of images I shows some images I_{L1}, I_{L2}, I_{Ln} out of a sequence S 1 captured from a segment 1 of live skin. The right column shows the images IF₁, I_{F2}, I_{Fn} out of another sequence S2 captured from a segment 1 of a fake finger, which can be a dismembered or cadaver finger or an imitation. Image I_{L1} shows four sweat droplets D₁ to D₄, which can be detected by segmenting the image I_{L1} and searching for spots whose colour or brightness significantly differs from the adjacent area. When using a 3D scanner the sweat droplets D₁ to D₄ can be identified by their 3D shape. It is critical that the optical sensor is touchless to allow sweat to emerge from the pores and to build droplets D instead of being smeared on a platen. Image I_{L2} is the subsequent image to I_{L1}. Apparently the size of the droplets D₁ to D₄ has changed compared to Image I_{L1}. Droplets D₂ and D₄ are grown indicating that sweat is still emerging from their pores. Droplets D₁ and D₃ are shrunk because their pores stopped sweating and the existing sweat started partially evaporating and partially getting absorbed. When comparing image I_{L1} with I_{L2}, sweat pore activity can easily be detected. The same applies to all subsequent images up to image I_{Ln}. In contrast neither one of the images I_{F1}, I_{F2}, I_{Fn} taken from the sequence S2 of the fake finger shows any sweat droplets D at all, hence there is no change between subsequent images I_{F1}, I_{F2}, I_{Fn}, which makes it easy to conclude that the finger is faked. Even if there were structures faking sweat droplets D on the surface of the finger there wouldn't be the same pattern of growing and shrinking droplets D observable like with perspiration on a live finger. The method can be applied at either ambient temperature conditions, which will normally be sufficient to show the sweating activity of live skin, or the sweating activity can be triggered by slightly heating the segment 1 of skin to observe the sweating activity as a response thereof, which is even harder to fake. Additionally the segment 1 can be illuminated in order to take full advantage of the optical sensor's dynamic range and to avoid noise in the image I. Different kinds of light sources can be used for that purpose, e.g. LED or fiber conductors with different spectral ranges (blue, green, yellow, red, infra red). Infra red light can serve for illumination and heating at the same time.

The skin segment 1 can be captured from any other part of the body, e.g. a palm.

The analysis for detecting sweat pore activity can comprise other steps than image segmentation.

Especially in case the segment 1 of skin is situated on a palm or finger, minutiae can be detected in the image I in order to combine liveness detection with fingerprint recognition or enrolling. The position of the sweat pores is unique and their detection can further improve fingerprint recognition and decrease false matching rate and false non-matching rate, especially when using a 3D scanner, which allows to locate the pores 3D position.

### List of References

1 segment of skin
S 1 sequence of images of live skin
S2 sequence of images of fake skin
I_{L1}, I_{L2}, I_{Ln} images of live skin
I_{F1}, I_{F2}, I_{Fn} images of fake skin
D₁ to D₄ sweat droplets

## Claims

1. Liveness detection method, wherein at least one image (I) of a segment (1) of human or animal skin is captured by a touchless optical sensor and subjected to an analysis for detecting a perspiration activity of sweat pores.

2. Liveness detection method according to claim 1, **characterized in, that** image segmentation is used for the analysis.

3. Liveness detection method according to one of the claims 1 or 2, **characterized in, that** a time sequence (S1, S2) of images (I) is captured and analyzed.

4. Liveness detection method according to one of the preceding claims, **characterized in, that** a change in color or brightness within the image and/or between at least two images is used for the detection of sweat pore perspiration activity.

5. Liveness detection method according to claim 4, **characterized in, that** said segment (1) of skin is heated to increase said sweat pore perspiration activity during capturing said sequence (S1, S2) of images (I).

6. Liveness detection method according to one of the preceding claims, **characterized in, that** the segment (1) of skin is illuminated.

7. Liveness detection method according to one of the preceding claims, **characterized in, that** minutiae are detected in said image (I).

8. Liveness detection method according to one of the preceding claims, **characterized in, that** the position of sweat pores is detected.

9. Liveness detection device, comprising at least a touchless optical sensor for capturing images (I) and an apparatus for detecting sweat pore perspiration activity in at least one of said images (I).

10. Liveness detection device according to claim 8, **characterized in, that** the touchless optical sensor is able to capture a 2D and/or 3D image (I).

11. Liveness detection device according to one of the claims 8 or 9, **characterized in, that** a heater is arranged so it can heat an area within the range of said touchless optical sensor.

12. Liveness detection device according to one of the claims 8 to 10, **characterized in, that** a light source is arranged so it can illuminate an area within the range of said touchless optical sensor.
